# EUROPÄISCHE PATENTSCHRIFT

(11) **EP 1 689 524 B1**
(45) Veröffentlichungstag und Bekanntmachung des Hinweises auf die Patenterteilung: **09.03.2011**
(21) Anmeldenummer: 04797918.2
(22) Anmeldetag: 16.11.2004
(51) Int. Cl.: B01J 23/888, C07C 45/35, C07C 47/22, B01J 35/02, B01J 35/00

(54) **VERFAHREN ZUR HERSTELLUNG VON ACROLEIN DURCH HETEROGEN KATALYSIERTE PARTIELLE GASPHASENOXIDATION VON PROPEN**
METHOD FOR THE PRODUCTION OF ACROLEIN BY THE HETEROGENEOUSLY-CATALYSED GAS-PHASE PARTIAL OXIDATION OF PROPENE
PROCEDE DE PRODUCTION D'ACROLEINE PAR OXYDATION PARTIELLE EN PHASE GAZEUSE A CATALYSE HETEROGENE DE PROPENE

(30) Priorität: 18.11.2003 US 520660 P; 18.11.2003 DE 10353954
(43) Veröffentlichungstag der Anmeldung: 16.08.2006
(73) Patentinhaber: BASF SE, 67056 Ludwigshafen (DE)
(72) Erfinder: PETZOLDT, Jochen, 67273 Weisenheim am Berg (DE); MÜLLER-ENGEL, Klaus Joachim, 76297 Stutensee (DE)
(86) Internationale Anmeldenummer: PCT/EP2004/012966
(87) Internationale Veröffentlichungsnummer: WO 2005/049200

(56) Entgegenhaltungen:
- EP-A- 0 575 897
- WO-A-02/24620

## Beschreibung

Vorliegende Erfindung betrifft ein Verfahren zur Herstellung von Acrolein durch heterogen katalysierte partielle Gasphasenoxidation, bei dem man ein Propen, molekularen Sauerstoff und wenigstens ein Inertgas enthaltendes Reaktionsgasausgangsgemisch, das den molekularen Sauerstoff und das Propen in einem molaren Verhältnis O₂ : C₃H₆ ≥ 1 sowie Kohlendioxid und gesättigte Kohlenwasserstoffe zusammen in einer Gesamtmenge von höchstens 15 mol-% enthält, bei erhöhter Temperatur und bei einer Belastung des Katalysatorfestbetts mit im Reaktionsgasausgangsgemisch enthaltenem Propen von ≥ 120 NI/l•h so durch ein Katalysatorfestbett, dessen Katalysatoren ringförmige Vollkatalysatoren sind, deren Aktivmasse wenigstens ein Multimetalloxid der allgemeinen Formel I,

Mo₁₂WₐCo_{b}Fe_{c}Bi_{d}SiₑK_{f}Oₙ (I),

ist, mit
- a =: ≥ 1 bis ≤ 3,
- b=: ≥ 3 bis ≤ 8,
- c =: ≥ 1 bis ≤ 4,
- d =: ≥ 0,5 bis ≤ 1,5,
- e =: ≥ 0 bis ≤ 10,
- f =: ≥ 0 bis ≤ 0,2 und
- n =: eine Zahl, die durch die Wertigkeit und Häufigkeit der von Sauerstoff verschiede- nen Elemente in I bestimmt wird,
führt, dass der Propenumsatz bei einmaligem Durchgang ≥ 90 mol-% und die damit einhergehende Selektivität der Acroleinbildung ≥ 80 mol-% betragen, dadurch gekennzeichnet dass Co/Fe = 2 bis 4 und Co/Mo = 0,5 bis 0,7.

Acrolein bildet ein reaktives Monomer, das insbesondere als Zwischenprodukt, z.B. bei der Herstellung von Acrylsäure durch zweistufige heterogen katalysierte partielle Gasphasenoxidation von Propen, von Bedeutung ist. Acrylsäure ist als solche oder in Form ihrer Alkylester z.B. zur Herstellung von Polymerisaten, die u.a. als Klebstoffe oder Wasser absorbierende Materialien Verwendung finden können, geeignet.

Die Herstellung von Acrolein durch das in der Präambel dieser Schrift beschriebene Verfahren der heterogen katalysierten partiellen Gasphasenoxidation ist bekannt (vgl. z.B. DE-A 10351269, DE-A 10350812, DE-A 10344149, DE-A 19948523, DE-A 10313209, DE-A 19948248, DE-A 19855913 und WO 02/24620). Üblicherweise bildet es die erste Stufe einer zweistufigen heterogen katalysierten Gasphasenpartialoxidation von Propen zu Acrylsäure. In der ersten Reaktionsstufe wird das Propen im wesentlichen partiell zu Acrolein oxidiert und in der zweiten Reaktionsstufe wird das in der ersten Reaktionsstufe gebildete Acrolein im wesentlichen partiell zu Acrylsäure oxidiert. Von Bedeutung ist dabei, dass die technische Ausführungsform normalerweise so ausgestaltet ist, dass das in der ersten Reaktionsstufe gebildete Acrolein nicht abgetrennt, sondern als Bestandteil des die erste Reaktionsstufe verlassenden Produktgasgemischs, gegebenenfalls durch molekularen Sauerstoff und Inertgas ergänzt, und gegebenenfalls durch direkte und/oder indirekte Kühlung abgekühlt, in die zweite Reaktionsstufe geführt wird.

Zielprodukt einer heterogen katalysierten partiellen Gasphasenoxidation von Propen zu Acrolein ist Acrolein.

Problempunkt aller heterogen katalysierten Gasphasenpartialoxidationen im Katalysatorfestbett ist, dass das Reaktionsgasgemisch beim Durchströmen durch das Katalysatorfestbett einen Höchstwert (den sogenannten Heißpunktwert) durchläuft.

Dieser Höchstwert setzt sich zusammen aus der externen Temperierung des Katalysatorfestbetts und der Reaktionswärme. Aus Gründen der Zweckmäßigkeit werden daher auch die Temperatur des Katalysatorfestbetts und die effektive Temperatur des Katalysatorfestbetts voneinander unterschieden. Dabei wird unter der Temperatur des Katalysatorbestbetts die Temperatur des Katalysatorfestbetts bei Ausübung des Partialoxidationsverfahrens jedoch in fiktiver Abwesenheit einer chemischen Reaktion (d.h., ohne den Einfluss der Reaktionswärme) verstanden. Unter effektiver Temperatur des Katalysatorfestbetts wird dagegen die tatsächliche Temperatur des Katalysatorfestbetts unter Einbezug der Reaktionswärme der Partialoxidation verstanden. Ist die Temperatur des Katalysatorfestbetts längs des Katalysatorfestbetts nicht konstant (z.B. im Fall von mehreren Temperaturzonen), so meint der Begriff Temperatur des Katalysatorfestbetts den (Zahlen)Mittelwert der Temperatur entlang des Katalysatorfestbetts. Von Bedeutung im vorgenannten Zusammenhang ist, dass die effektive Temperatur des Katalysatorfestbetts mit der Temperatur des Reaktionsgasgemischs in Strömungsrichtung des Reaktionsgasgemischs gleichfalls den Heißpunktwert durchläuft.

Nachteilig an den bekannten Verfahren gemäß der Präambel dieser Schrift ist, dass bei vorgegebenem Umsatz des Propens (bezogen auf einmaligen Durchgang des Reaktionsgasgemischs), die mit den im Stand der Technik empfohlenen Katalysatoren einhergehenden Heißpunkttemperaturen des Katalysatorfestbetts bei den geforderten Propenbelastungen des Katalysatorfestbetts zu hoch sind (hohe Heißpunkttemperaturen sind normalerweise insofern von Nachteil, als hohe Temperaturen einerseits den Alterungsprozess des Katalysatorfestbetts beschleunigen (bestimmte Bewegungsprozesse innerhalb der Aktivmasse der Katalysatoren, die zur Alterung beitragen, laufen z.B. schneller ab) und andererseits die Selektivität der Zielproduktbildung mindern), weshalb im Stand der Technik die Katalysatoren in der Regel nach speziellen Verdünnungsprofilen in notwendiger Weise mit Inertmaterial verdünnt im Katalysatorfestbett vorliegen. Letzteres begrenzt bei vorgegebenem Umsatz des Propens aber die mögliche Belastung des Katalysatorfestbetts mit im Reaktionsgasausgangsgemisch enthaltenem Propen.

Die Aufgabe der vorliegenden Erfindung bestand daher darin, ein wie eingangs dieser Schrift beschriebenes Verfahren zur Verfügung zu stellen, wobei die Katalysatoren jedoch so ausgewählt sind, dass insbesondere bei hoher Propenbelastung des Katalysatorfestbetts der vorgegebene Propenumsatz bei verminderter Heißpunkttemperatur des Katalysatorfestbetts und erhöhter Zielproduktselektivität erreicht wird.

Demgemäss wurde ein Verfahren zur Herstellung von Acrolein durch heterogen katalysierte partielle Gasphasenoxidation, bei dem man ein Propen, molekularen Sauerstoff und wenigstens ein Inertgas enthaltendes Reaktionsgasausgangsgemisch, das den molekularen Sauerstoff und das Propen in einem molaren Verhältnis O₂: C₃H₆ ≥ 1 sowie Kohlendioxid und gesättigte Kohlenwasserstoffe zusammen in einer Gesamtmenge von höchstens 15 mol% enthält, bei erhöhter Temperatur und bei einer Belastung des Katalysatorfestbetts mit im Reaktionsgasausgangsgemisch enthaltenem Propen von ≥ 120 NI/I•h so durch ein Katalysatorfestbett, dessen Katalysatoren ringförmige Volikatalysatoren sind, deren Aktivmasse wenigstens ein Multimetalloxid der allgemeinen Formel I,

Mo₁₂WₐCo_{b}Fe_{c}Bi_{d}SiₑK_{f}Oₙ (I),

ist, mit
- a =: ≥ 1 bis ≤ 3,
- b =: ≥ 3 bis ≤ 8,
- c =: ≥ 1 bis ≤ 4,
- d =: ≥ 0,5 bis ≤ 1,5,
- e=: ≥ 0 bis ≤ 10,
- f =: ≥ 0 bis ≤ 0,2 und
- n =: eine Zahl, die durch die Wertigkeit und Häufigkeit der von Sauerstoff verschiede- nen Elemente in I bestimmt wird,
führt, dass der Propenumsatz bei einmaligem Durchgang ≥ 90 mol-% und die damit einhergehende Selektivität der Acroleinbildung ≥ 80 mol% betragen, gefunden, das dadurch gekennzeichnet ist, dass in der Multimetalloxidmasse I zusätzlich die nachfolgenden molaren Verhältnisse erfüllt sind: Co/Fe = 2 bis 4 und Co/Mo = 0.5 bis 0,7.

Multimetalloxid I - Katalysatoren bei denen die vorgenannten Verhältnisse erfüllt sind, wurden bereits in der EP-A 293224 für die heterogen katalysierte Partialoxidation von Propen zu Acrolein unter hoher Propenbelastung des Katalysatorfestbetts eingesetzt. Dies allerdings unter der Voraussetzung, dass der Gehalt des Reaktionsgasausgangsgemischs an Kohlendioxid und gesättigten Kohlenwasserstoffen zusammen ≥ 20 Vol.% beträgt (unter diesen Bedingungen ist das erfindungsgemäße Verfahren in nicht erfindungsgemäßer Weise selbstredend auch ausführbar).

Aus der DE-A 19948523 ist bekannt, dass die ringförmige Katalysatorgeometrie für heterogen katalysierte Partialoxidationen mit hoher Reaktandenbelastung des Katalysatorfestbetts besonders vorteilhaft ist.

Erfindungsgemäß bevorzugt beträgt der stöchiometrische Koeffizient a 1,5 bis 2,5 und besonders bevorzugt beträgt er 2 bis 2,5.

Der stöchiometrische Koeffizient b ist erfindungsgemäß bevorzugt ≥ 4 und ≤ 8, besonders bevorzugt ≥ 5 und ≤ 8 und ganz besonders bevorzugt ≥ 6 und ≤ 8 bzw. ≥ 6,5 und ≤ 7,5.

Der stöchiometrische Koeffizient c beträgt erfindungsgemäß vorteilhaft ≥ 2 bis ≤ 4 und besonders vorteilhaft ≥ 2,5 bis ≤ 3,5.

Ferner ist es für das erfindungsgemäße Verfahren günstig, wenn der stöchiometrische Koeffizient d 1 bis 1,5 beträgt.

Auch ist es für das erfindungsgemäße Verfahren von Vorteil, wenn der stöchiometrische Koeffizient e ≥ 0,5 bis ≤ 3, und von besonderem Vorteil wenn c ≥ 1 bzw. ≥ 1,5 und ≤ 2,5 bzw. ≤ 2 ist.

Der stöchiometrische Koeffizient f liegt vorzugsweise im Bereich ≥ 0,01 bis ≤ 0,15 und ganz besonders bevorzugt im Bereich ≥ 0,05 bis ≤ 0,1.

Die vorgenannten Vorzugsbereiche gelten zunächst einmal unabhängig voneinander. Besonders vorteilhaft sind jedoch mehrere von ihnen (und ganz besonders vorteilhaft alle von ihnen) gleichzeitig gegeben.

Unabhängig von den vorgenannten Vorzugsbereichen ist es für das erfindungsgemäße Verfahren vorteilhaft, wenn das molare Verhältnis Co/Fe 2 bis 3,5, oder besser 2 bis 3, oder noch besser 2 bis 2,5 beträgt.

Weiterhin ist es für das erfindungsgemäße Verfahren unabhängig von den bereits genannten Vorzugsbereichen günstig, wenn das molare Verhältnis Co/Mo 0,5 bis 0,7 bzw. bis 0,6 und am besten 0,55 bis 0,7 bzw. bis 0,6 beträgt.

Besonders vorteilhaft befinden sich, unabhängig von den sonstigen Vorzugsbereichen für die stöchiometrischen Koeffizienten a bis f, die beiden vorgenannten molaren Verhältnisse Co/Fe und Co/Mo gleichzeitig in den vorgenannten Vorzugsbereichen. Am besten befinden sich sowohl die stöchiometrischen Koeffizienten a bis f, als auch die molaren Verhältnisse Co/Fe und Co/Mo in ihren Vorzugsbereichen.

Erfindungsgemäß wesentlich ist, dass die Katalysatoren des Katalysatorfestbetts ringförmige Vollkatalysatoren sind. D.h., die Aktivmasse befindet sich nicht auf einem inerten Trägerkörper aufgetragen, sondern der gesamte ringförmige Katalysatorformkörper besteht aus katalytisch aktivem Multimetalloxid I.

Erfindungsgemäß vorteilhaft weist ihre ringförmige Geometrie (wie immer in dieser Schrift ohne Berücksichtigung einer gegebenenfalls bestehenden Krümmung der Stirnfläche) eine Länge L von 2 bis 11 mm, einen Außendurchmesser A von 2 bis 11 mm und eine Wandstärke W von 0,5 bis 5 mm auf.

Im Fall von erfindungsgemäß geeigneten Vollkatalysatorringen kommen ferner insbesondere jene in Betracht, für die gilt, dass der Innendurchmesser I das 0,1- bis 0,7-fache des Außendurchmessers und die Länge das 0,5- bis 2-fache des Außendurchmessers beträgt.

Günstige erfindungsgemäß verwendbare Vollkatalysatorringe sind auch solche mit einem Außendurchmesser von 2 bis 10 mm (bzw. 3 bis 7 mm), einem Ringinnendurchmesser von wenigstens 1 mm, einer Wanddicke von 0,5 bis 2 mm (bzw. 0,75 mm bis 1,75 mm) und einer Länge (Höhe) von 2 bis 10 mm (bzw. 2 bis 5 mm).

Häufig wird bei erfindungsgemäß ganz besonders geeigneten Vollkatalysatoren der Außendurchmesser 5,5 bis 7 mm, die Höhe (Länge) 2,8 bis 3,2 mm und der Innendurchmesser 3,5 bis 5 mm betragen. Typische Wandstärken liegen dabei bei 1,0 bis 1,7 mm, vorzugsweise bei 1,3 bis 1,7 mm und ganz besonders bevorzugt bei 1,5 mm.

D.h., erfindungsgemäß geeignete Hohlzylindervollkatalysatorgeometrien sind z.B. (jeweils Außendurchmesser x Höhe x Innendurchmesser, und wie immer in dieser Schrift ohne Berücksichtigung einer gegebenenfalls bestehenden Krümmung der Stirnfläche) die Geometrien 6 mm x 3 mm x 4 mm, 5 mm x 3 mm x 2 mm, 6,5 mm x 3 mm x 4,5 mm, 5 mm x 2 mm x 2 mm, 5 mm x 3 mm x 3 mm, 6 mm x 3 mm x 3mm, 7 mm x 3 mm x 4 mm, 7 mm x 3 mm x 5 mm sowie 5,5 mm x 3 mm x 3,5 mm.

Die Stirnflächen der erfindungsgemäß geeigneten Katalysatorringgeometrien können auch entweder beide oder nur eine wie in der EP-A 184790 beschrieben gekrümmt sein und zwar z.B. so, dass der Radius der Krümmung vorzugsweise das 0,4- bis 5-fache des Außendurchmessers beträgt. Erfindungsgemäß bevorzugt sind beide Stirnflächen ungekrümmt.

Die Herstellung der erfindungsgemäß zu verwendenden ringförmigen Vollkatalysatoren kann in einfacher Weise dadurch erfolgen, dass man aus Quellen der elementaren Konstituenten der Aktivmasse ein feinteiliges formbares Gemisch erzeugt und aus diesem Gemisch, gegebenenfalls nach Zugabe von Formungs- und/oder Verstärkungshilfsmitteln, ringförmige Vollkatalysatorvorläuferformkörper formt, deren Stirnflächen gekrümmt und/oder nicht gekrümmt sind, und diese durch thermisches Behandeln bei erhöhter Temperatur in die gewünschten Vollkatalysatoren überführt.

Erfindungsgemäß vorteilhaft beträgt dabei die Seitendruckfestigkeit der ringförmigen Vollkatalysatorvorläuferformkörper ≥ 10 N und ≤ 25 N, besonders vorteilhaft ≥ 12 N und ≤ 23 N bzw. ≥ 13 N und ≤ 22 N sowie ganz besonders vorteilhaft ≥ 14 N und ≤ 21 N bzw. ≥ 15 N und ≤ 20 N.

Ferner beträgt die Körnung des zu ringförmigen Vollkatalysatorvorläuferformkörpern zu formenden feinteiligen formbaren Gemischs erfindungsgemäß vorteilhaft 200 µm bis 1,5 mm, besonders vorteilhaft 400 µm bis 1 mm. In günstiger Weise liegen wenigstens 95 oder 98 oder mehr Gew.-% der Gesamtmasse in diesem Körnungsbereich.

Als Seitendruckfestigkeit wird dabei in dieser Schrift die Druckfestigkeit bei Stauchung des ringförmigen Vollkatalysatorvorläuferformkörpers senkrecht zur zylindrischen Einhüllenden (d.h., parallel zur Fläche der Ringöffnung) verstanden.

Dabei beziehen sich alle Seitendruckfestigkeiten dieser Schrift auf eine Bestimmung mittels einer Material-Prüfmaschine der Firma Zwick GmbH & Co. (D-89079 Ulm) des Typs Z 2.5/TS1 S. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500 N einsetzbar (relative Messunsicherheit: ± 0,2 %).

Die Messungen wurden mit folgenden Parametern durchgeführt:
Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor die Fläche der zylindrischen Einhüllenden des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der ringförmige Vollkatalysatorvorläuferformkörper Rissbildung zeigt, ist die Seitendruckfestigkeit (SDF).

Als Formungshilfsmittel (Gleitmittel) kommen zur Herstellung der ringförmigen Vollkatalysatorvorläuferformkörper z.B. Ruß, Stearinsäure, Stärke, Polyacrylsäure, Mineral- oder Pflanzenöl, Wasser, Bortrifluorid oder Graphit in Betracht. Auch Glycerin und Celluloseether können als Gleitmittel eingesetzt werden. Bezogen auf die zum Vollkatalysatorvorläuferformkörper zu formende Masse werden in der Regel ≤ 5 Gew.-%, meist ≤ 3 Gew.%, vielfach ≤ 2 Gew.-% an Formungshilfsmittel zugesetzt. Üblicherweise beträgt die vorgenannte Zusatzmenge ≥ 0,5 Gew.-%. Erfindungsgemäß bevorzugtes Gleithilfsmittel ist Graphit.

Im Rahmen der thermischen Behandlung der ringförmigen Vollkatalysatorvorläuferformkörper werden die Formungshilfsmittel meist weitgehend in gasförmige Komponenten zersetzt und/oder verbrannt, so dass der erhaltene ringförmige Vollkatalysator normalerweise teilweise oder vollständig frei von mitverwendeten Formungshilfsmitteln ist. Soweit noch Formungshilfsmittel in den erfindungsgemäß erhältlichen ringförmigen Vollkatalysatoren enthalten ist, verhält es sich bezüglich der durch die Vollkatalysatoren katalysierten Partialoxidation von Propen zu Acrolein im wesentlichen inert.

Letzteres gilt auch für gegebenenfalls vorab der Formgebung zugesetzte feinteilige Verstärkungsmittel wie Mikrofasern aus Glas, Asbest, Siliciumcarbid oder Kaliumtitanat. Die Formung zum ringförmigen Vollkatalysatorvorläuferformkörper kann z.B. mittels einer Tablettierungsmaschine, einer Extrusionsverformungsmaschine, einer Strangpressvorrichtung oder dergleichen durchgeführt werden.

Die thermische Behandlung des ringförmigen Vollkatalysatorvorläuferformkörpers erfolgt in der Regel bei Temperaturen, die 350°C überschreiten. Normalerweise wird im Rahmen der thermischen Behandlung die Temperatur von 650°C jedoch nicht überschritten. Erfindungsgemäß vorteilhaft wird im Rahmen der thermischen Behandlung die Temperatur von 600°C, bevorzugt die Temperatur von 550°C und besonders bevorzugt die Temperatur von 500°C nicht überschritten. Ferner wird beim erfindungsgemäßen Verfahren im Rahmen der thermischen Behandlung des ringförmigen Vollkatalysatorvorläuferformkörpers vorzugsweise die Temperatur von 380°C, mit Vorteil die Temperatur von 400°C, mit besonderem Vorteil die Temperatur von 420°C und ganz besonders bevorzugt die Temperatur von 440°C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350°C, vorzugsweise 220 bis 280°C, und daran anschließend eine thermische Behandlung bei einer Temperatur von 400 bis 600°C, vorzugsweise 430 bis 550°C durchgeführt werden.

Normalerweise nimmt die thermische Behandlung des ringförmigen Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringförmigen Vollkatalysatorvorläuferformkörpers Behandlungsdauern von 45 h bzw. 25 h nicht überschritten. Oft liegt die Gesamtbehandlungsdauer unterhalb von 20 h. Erfindungsgemäß vorteilhaft werden im Rahmen der thermischen Behandlung des ringförmigen Vollkatalysatorvorläuferformkörpers 500°C (460°C) nicht überschritten und die Behandlungsdauer im Temperaturfenster von ≥ 400°C (≥ 440°C) erstreckt sich auf 5 bis 20 h.

Die thermische Behandlung (auch die nachfolgend angesprochene Zersetzungsphase) der ringförmigen Vollkatalysatorvorläuferformkörper kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂ oder Methan, Acrolein, Methacrolein) erfolgen. Selbstredend kann die thermische Behandlung auch unter Vakuum ausgeführt werden.

Prinzipiell kann die thermische Behandlung der ringförmigen Vollkatalysatorvorläuferformkörper in den unterschiedlichsten Ofentypen wie z.B. beheizbare Umluftkammern, Hordenöfen, Drehrohröfen, Bandcalzinierer oder Schachtöfen durchgeführt werden. Erfindungsgemäß bevorzugt erfolgt die thermische Behandlung der ringförmigen Vollkatalysatorvorläuferformkörper in einer Bandcalciniervorrichtung, wie sie die DE-A 10046957 und die WO 02/24620 empfehlen.

Die thermische Behandlung der ringförmigen Vollkatalysatorvorläuferformkörper unterhalb von 350°C verfolgt in der Regel die thermische Zersetzung der in den Vollkatalysatorvorläuferformkörpern enthaltenen Quellen der elementaren Konstituenten des angestrebten ringförmigen Vollkatalysators. Häufig erfolgt beim erfindungsgemäßen Verfahren diese Zersetzungsphase im Rahmen des Aufheizens auf Temperaturen ≥ 350°C.

Die ringförmigen Vollkatalysatorvorläuferformkörper können, wie bereits erwähnt, dadurch hergestellt werden, dass man von Quellen der elementaren Konstituenten der Aktivmasse des gewünschten ringförmigen Vollkatalysators ein (möglichst inniges) feinteiliges, der Stöchiometrie der gewünschten Aktivmasse entsprechend zusammengesetztes, formbares Gemisch erzeugt und aus diesem, gegebenenfalls nach Zusatz von Formungs- und/oder Verstärkungshilfsmitteln, einen ringförmigen Vollkatalysatorvorläuferformkörper (mit gekrümmter und/oder nicht gekrümmter Stirnfläche) formt (dessen Seitendruckfestigkeit vorteilhaft ≥ 10 N und ≤ 25 N beträgt; sie kann aber auch 1 N bis < 10 N, d.h., z.B. 4 N bis 8 N betragen). Die Geometrie des ringförmigen Vollkatalysatorvorläuferformkörpers wird dabei im wesentlichen derjenigen des gewünschten ringförmigen Vollkatalysators entsprechen.

Als Quellen für die elementaren Konstituenten der gewünschten Aktivmasse kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu vollständig gasförmig entweichenden Verbindungen zerfallen und/oder zersetzt werden können, können in das feinteilige formbare Gemisch (vorzugsweise ein Trockengemisch) zusätzlich eingearbeitet werden).

Das, vorzugsweise innige, Vermischen der Ausgangsverbindungen (Quellen) zur Herstellung des feinteiligen formbaren Gemischs kann beim erfindungsgemäßen Verfahren in trockener oder in nasser Form erfolgen. Erfolgt es in trockener Form, so werden die Ausgangsverbindungen zweckmäßigerweise als feinteilige Pulver (die Körnung sollte vorteilhaft ≤ 100 µm, vorzugsweise ≤ 50 µm betragen; in der Regeln wird der zahlenmittlere Korngrößtdurchmesser ≥ 10 µm betragen) eingesetzt. Nach eventuellem Zusatz von Formungs- und/oder Verstärkungshilfsmitteln kann anschließend die Formgebung zum ringförmigen Vollkatalysatorvorläuferformkörper erfolgen.

Erfindungsgemäß bevorzugt erfolgt das innige Vermischen jedoch in nasser Form. Üblicherweise werden dabei die Ausgangsverbindungen in Form einer wässrigen Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene Lösung oder Suspension getrocknet, wobei der Trocknungsprozess vorzugsweise durch Sprühtrocknung mit Austrittstemperaturen von 100 bis 150°C erfolgt. Die Körnung des resultierenden Sprühpulvers beträgt in typischer Weise 20 bis 50 µm.

Das Sprühpulver kann nun als solches oder nach Zusatz von Formungs- und/oder Verstärkungshilfsmittel zu den ringförmigen Vollkatalysatorvorläuferformkörpern verdichtet (geformt) werden. Die feinteiligen Verstärkungshilfsmittel können aber auch bereits vorab der Sprühtrocknung (teilweise oder vollständig) zugesetzt werden. Auch kann bei der Trocknung das Lösungs- bzw. Suspendiermittel nur teilweise entfernt werden, falls seine Mitverwendung als Formungshilfsmittel beabsichtigt ist.

Anstatt das Sprühpulver, gegebenenfalls nach Zusatz von Formungs- und/oder Verstärkungshilfsmittel, unmittelbar zu den ringförmigen Vollkatalysatorvorläuferformkörpern (mit gekrümmter und/oder nicht gekrümmter Stirnfläche der Ringe) zu formen, ist es häufig zweckmäßig, zunächst eine Zwischenkompaktierung durchzuführen, um das Pulver zu vergröbern (in der Regel auf eine Körnung von 400 µm bis 1 mm). Anschließend erfolgt mit dem vergröberten Pulver die eigentliche Ringformung, wobei bei Bedarf zuvor nochmals feinteiliges Gleitmittel zugegeben werden kann.

Als günstiges Gleitmittel für eine solche Zwischenkompaktierung (ebenso wie für die Endformung) hat sich feinteiliges Graphit der Fa. Timcal AG (San Antonio, US) vom Typ TIMREX P44 bzw. Graphitpulver T44 der Fa. Lonza, CH-5643 Sins (Siebanalyse oder Laserbeugung: min. 50 Gew.-% < 24 µm, max. 10 Gew.% > 24 µm und ≤ 48 µm, max. 5 Gew.-% > 48 µm, BET-Oberfläche: 6 bis 13 m²/g) als zweckmäßig erwiesen. Es fungiert nach der durchgeführten Zwischenkompaktierung gleichzeitig als Gleitmittel bei der eigentlichen Ringformung (und kann bei Bedarf wie beschrieben zuvor zusätzlich ergänzt werden). Es erweist sich als günstig, wenn der Ascherückstand des verwendeten Graphit (Glühen bei 815°C unter Luft) ≤ 0,1 Gew.-% beträgt.

Eine solche Zwischenkompaktierung zum Zweck der Kornvergröberung kann beispielsweise mittels eines Kompaktierers der Fa. Hosokawa Bepex GmbH (D-74211 Leingarten), vom Typ Kompaktor K 200/100 erfolgen. Die Härte des Zwischenkompaktats liegt häufig bereits im Bereich von 10 N. Für die Ringformung zum Vollkatalysatorvorläuferformkörper kommt z.B. ein Kilian Rundläufer (der Fa. Kilian in D-50735 Köln) vom Typ RX 73 oder S 100 in Betracht. Alternativ kann eine Tablettenpresse der Fa. Korsch (D-13509 Berlin) vom Typ PH 800-65 eingesetzt werden.

Bei der Herstellung der ringförmigen Vollkatalysatoren ist es vorteilhaft (vgl. DE-A 4407020, EP-A 835, EP-A 575897, DE-C 3338380, DE-A 10344149), zur Herstellung des ringförmigen Vollkatalysatorvorläuferformkörpers als Quelle der Elemente W und Bi in Abwesenheit der übrigen Konstituenten der Aktivmasse I ein Mischoxid II,

Bi_{a'}W_{b'}O_{x'} (II),

mit
- a' =: 0,01 bis 8,
- b' =: 0,1 bis 30 und
- x' =: eine Zahl, die durch die Wertigkeit und Häufigkeit der von Sauerstoff verschiede- nen Elemente in II bestimmt wird,
vorzubilden, und damit nach seiner Vorbildung, wie bereits beschrieben, mit Quellen der übrigen Konstituenten der gewünschten Aktivmasse I ein feinteiliges formbares Gemisch zu erzeugen, um daraus, gegebenenfalls noch Zusatz von Formungs- und/oder Verstärkungshilfsmitteln, den ringförmigen Vollkatalysatorvorläuferformkörper zu formen. Bei einer solchen Vorgehensweise hat es sich als vorteilhaft erwiesen, wenn für den Fall, dass die Herstellung des feinteiligen formbaren Gemischs nass erfolgt (in Suspension), das vorgebildete Mischoxid Bi_{a'}W_{b'}O_{x'} nicht in nennenswertem Umfang in Lösung geht.

Bevorzugte Mischoxide II sind solche der Stöchiometrie BiWO₆, Bi₂W₂O₉ sowie Bi₂W₃O₁₂.

Selbstverständlich kann nur für eine Teilmenge (z.B. 25 mol-%, oder 50 mol-% oder 75 mol-% der jeweiligen Gesamtmenge) des im Multimetalloxid I enthaltenen W und Bi ein Mischoxid II als Quelle verwendet werden. Vorzugsweise wird jedoch für die Gesamtmenge des im Multimetalloxid I enthaltenen W und Bi als Quelle ein Mischoxid II verwendet.

Die Herstellung von Mischoxiden II kann dabei z.B. wie folgt erfolgen. Wasserlösliche Salze des Bi wie z.B. dessen Nitrate, Carbonate, Hydroxide oder Acetate werden mit W-Säuren oder deren Ammoniumsalzen in Wasser gemischt, die Mischung getrocknet (vorzugsweise sprühgetrocknet) und die getrocknete Masse anschließend thermisch behandelt.

Die thermisch behandelte Masse wird nachfolgend zweckmäßig zerkleinert (z.B. in einer Kugelmühle oder durch Strahlmahlen) und aus dem dabei erhältlichen, in der Regel aus im wesentlichen kugelförmigen Partikeln bestehenden, Pulver die Kornklasse mit einem im für die Aktivmasse I gewünschten Größtdurchmesserbereich liegenden Korngrößtdurchmesser (z.B. 1 nm bis 100 µm, häufig 10 nm bis 500 nm oder 1 µm bis 50 bzw. 25 µm) durch in an sich bekannter Weise durchzuführendes Klassieren (z.B. Nass- oder Trockensiebung) abgetrennt. Die thermische Behandlung im Rahmen der Herstellung von Mischoxiden II erfolgt zweckmäßig bei Temperaturen von 400 bis 900°C, vorzugsweise bei 600 bis 900°C. Üblicherweise erfolgt die thermische Behandlung im Luftstrom (z.B. in einem Drehrohrofen, wie er in der DE-A 103 25 487 beschrieben ist). Die Dauer der thermischen Behandlung erstreckt sich in der Regel auf wenige Stunden.

Erfindungsgemäß bevorzugt wird nicht das feinteilige Mischoxid II als solches als Quelle zur Herstellung eines Multimetalloxids I eingesetzt. Erfindungsgemäß zweckmäßig mischt man dem feinteiligen Mischoxid II bevorzugt (bezogen auf die Masse des feinteiligen Mischoxids) 0,1 bis 3 Gew.-% feinteiliges SiO₂ (der zahlenmittlere Korngrößtdurchmesser der üblicherweise im wesentlichen kugelförmigen SiO₂-Partike beträgt zweckmäßigerweise 10 bis 50 nm) zu und stellt so eine Ausgangsmasse 1 her, die als eigentliche Quelle verwendet wird.

Von den übrigen Bestandteilen der gewünschten aktiven Multimetalloxidmasse I wird dann normalerweise ausgehend von in an sich bekannter Weise geeigneten Quellen (vgl. EP-A 835 und DE-C 333 8380 sowie DE-A 4407020) in erfindungsgemäß zweckmäßiger Weise z.B. ein möglichst inniges, vorzugsweise feinteiliges Trockengemisch hergestellt (z.B. wasserlösliche Salze wie Halogenide, Nitrate, Acetate, Carbonate oder Hydroxide in einer wässrigen Lösung vereinen und anschließend die wässrige Lösung z.B. sprühtrocknen oder nicht wasserlösliche Salze, z.B. Oxide, in wässrigem Medium suspendieren und anschließend die Suspension z.B. sprühtrocknen), das hier als Ausgangsmasse 2 bezeichnet wird. Wesentlich ist nur, dass es sich bei den Bestandteilen der Ausgangsmasse 2 entweder bereits um Oxide handelt, oder um solche Verbindungen, die durch Erhitzen, gegebenenfalls in Anwesenheit von Sauerstoff, in Oxide überführbar sind. Anschließend werden die Ausgangsmasse 1 und die Ausgangsmasse 2 im gewünschten Mengenverhältnis, gegebenenfalls nach Zusatz von Formungs- und/oder Verstärkungshilfsmittein, zum zum ringförmigen Vollkatalysatorvorläuferformkörper formbaren Gemisch vermischt. Die Formung kann, wie bereits beschrieben, anwendungstechnisch zweckmäßig über die Stufe einer Zwischenkompaktierung erfolgen.

In einer weniger bevorzugten Ausführungsform kann das vorgebildete Mischoxid II mit Quellen der übrigen Bestandteile der gewünschten Aktivmasse I auch in flüssigem, vorzugsweise wässrigem, Medium innig vermischt werden. Dieses Gemisch wird anschließend z.B. zu einem innigen Trockengemisch getrocknet und sodann, wie bereits beschrieben, geformt und thermisch behandelt. Dabei können die Quellen der übrigen Konstituenten in diesem flüssigen Medium gelöst und/oder suspendiert vorliegen, wohingegen das vorgebildete Mischoxid II in diesem flüssigen Medium im wesentlichen unlöslich sein sollte, d.h., suspendiert vorliegen muss.

Die vorgebildeten Mischoxid-II-partikel sind im fertiggestellten ringförmigen Vollkatalysator normalerweise in der durch die Klassierung eingestellten Längstausdehnung im wesentlichen unverändert enthalten.

D.h., es sind so Multimetalloxide I erhältlich, die z.B. wenigstens 25 mol.-% (bevorzugt wenigstens 50 mol.-% und besonders bevorzugt 100 mol.-%) des in ihnen insgesamt enthaltenen W in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Bi_{a'}W_{b'}O_{x'}, deren Größtdurchmesser vorteilhaft im Bereich von 1 nm bis 100 µm liegt, enthalten.

Erfindungsgemäß bevorzugt beträgt die spezifische Oberfläche von solchermaßen vorgebildeten Mischoxiden II 0,2 bis 2, vorzugsweise 0,5 bis 1,2 m²/g. Ferner resultiert das Porengesamtvolumen von solchermaßen vorgebildeten Mischoxiden II vorteilhaft überwiegend von Mikroporen.

Alle Angaben in dieser Schrift zu Bestimmungen von spezifischen Oberflächen bzw. von Mikroporenvolumina beziehen sich auf Bestimmungen nach DIN 66131 (Bestimmung der spezifischen Oberfläche von Feststoffen durch Gasadsorption (N₂) nach Brunauer-Emmet-Teller (BET)).

Alle Angaben in dieser Schrift zu Bestimmungen von Porengesamtvolumina sowie von Durchmesserverteilungen auf diese Porengesamtvolumina beziehen sich, soweit nichts anderes erwähnt wird, auf Bestimmungen mit der Methode der Quecksilberporosimetrie in Anwendung des Gerätes Auto Pore 9220 der Fa. Micromeritics GmbH, 4040 Neuß, DE (Bandbreite 30Å bis 0,3 mm).

Prinzipiell sind die erfindungsgemäß zu verwendenden ringförmigen Vollkatalysatoren auch nach den in den Schriften WO 03/039744, EP-A 279374 und EP-A 1340538 beschriebenen Herstellverfahren erhältlich.

In entsprechender Weise können sie auch die in diesen Schriften empfohlenen physikalischen Eigenschaften (spezifische Oberfläche, Porengesamtvolumen, Beitrag der einzelnen Porendurchmesser zum Porengesamtvolumen, Verhältnis R (R = 1/(1+V•p), mit V = Porengesamtvolumen) von scheinbarer Massendichte zu wahrer Massendichte p etc.) aufweisen.

D.h., R kann für die erfindungsgemäß zu verwendenden ringförmigen Vollkatalysatoren sowohl 0,25 bis 0,55, als auch > 0,55 betragen. Bevorzugt beträgt R ≤ 0,9 bzw. ≤ 0,8 und ≥ 0,6 bzw. ≥ 0,65.

Erfindungsgemäß vorteilhaft zu verwendende ringförmige Vollkatalysatoren sind ferner solche, deren spezifische Oberfläche O 5 bis 20 bzw. 15 m²/g, häufig 5 bis 10 m²/g beträgt. Das Porengesamtvolumen V von erfindungsgemäß besonders geeigneten ringförmigen Vollkatalysatoren liegt vorteilhaft im Bereich von 0,1 bis 1 bzw. 0,8 cm³/g, häufig im Bereich 0,2 bis 0,4 cm³/g.

In vorteilhafter Weise tragen die verschiedenen Porendurchmesser bei erfindungsgemäß einzusetzenden ringförmigen Vollkatalysatoren in vorteilhafter Weise wie folgt zum Porengesamtvolumen (Porenverteilung A) bei:
Poren mit Durchmesser im Bereich < 0,03 µm: ≤ 5 Vol.-%;
Poren mit Durchmesser im Bereich von ≥ 0,03 bis ≤ 0,1 µm: ≤ 25 Vol.-%;
Poren mit Durchmesser im Bereich von > 0,1 bis < 1 µm 70 Vol.-%; und
Poren mit Durchmesser im Bereich ≥ 1 bis ≤ 10 µm: ≤ 10 Vol.-%.

Besonders vorteilhaft verteilt sich bei erfindungsgemäß zu verwendenden ringförmigen Vollkatalysatoren der Anteil der verschiedenen Porendurchmesser am Porengesamtvolumen wie folgt (Porenverteilung B):
Poren mit Durchmesser im Bereich < 0,03 µm 0 und ≤ 5 Vol.-%, vorzugsweise ≤ 3 Vol.-%;
Poren mit Durchmesser im Bereich von ≥ 0,03 bis ≤ 0,1 µm 3 bzw. ≥ 5 und ≤ 20 bzw. ≤ 15 Vol.-%;
Poren mit Durchmesser im Bereich von > 0,1 bis < 1 µm 75 bzw. ≥ 80 und ≤ 95 bzw. ≤ 90 Vol.-%;
Poren mit Durchmesser im Bereich ≥ 1 µm bis ≤ 10 µm 0 und ≤ 5 Vol.-%, vorzugsweise ≤ 3 Vol.-%.

Ferner ist es für erfindungsgemäß zu verwendende ringförmige Vollkatalysatoren günstig, wenn derjenige Porendurchmesser, der prozentual den größten Beitrag zum Porengesamtvolumen V leistet, d^{max}, 0,3 bis 0,8 µm, besonders vorteilhaft 0,4 bis 0,7 µm und ganz besonders vorteilhaft 0,5 bis 0,6 µm beträgt.

Besonders bevorzugt werden für das erfindungsgemäße Verfahren solche ringförmigen Vollkatalysatoren eingesetzt, für die simultan gilt:
- O =: 5 bis 10 m²/g, vorzugsweise 5 bis 15 m²/g, besonders bevorzugt 5 bis 10 m²/g;
- V =: 0,1 bis 1 cm³/g, vorzugsweise 0,1 bis 0,8 cm³/g, besonders bevorzugt 0,2 bis 0,4 cm³/g; und
Porenverteilung = Porenverteilung A oder Porenverteilung B.

Weiterhin ist es erfindungsgemäß vorteilhaft wenn gleichzeitig erfüllt ist:
- d^{max} =: 0,3 bis 0,8 µm, vorzugsweise 0,4 bis 0,7 µm und besonders bevorzugt 0,5 bis 0,6 µm.

In erfindungsgemäß bemerkenswerter Weise fördern Poren im Porendurchmesserbereich > 0,1 bis < 1 µm insbesondere die Selektivität der Acroleinbildung.

Im Unterschied dazu fördern Poren im Porendurchmesserbereich von 0,01 bis 0,1 µm eher die Selektivität der Acrylsäurenebenproduktbildung.

Mit zunehmender Seitendruckfestigkeit des ringförmigen Vollkatalysatorvorläuferformkörpers werden die Porendurchmesser im resultierenden Vollkatalysatorring in der Regel zu größeren Werten verschoben. Dabei verschiebt sich gleichzeitig die Seitendruckfestigkeit des resultierenden ringförmigen Vollkatalysators normalerweise zu höheren Werten. Die Seitendruckfestigkeit des resultierenden Vollkatalysators ist im Regelfall kleiner als die Seitendruckfestigkeit des zugehörigen ringförmigen Vollkatalysatorvorläuferformkörpers.

In typischer Weise betragen die Seitendruckfestigkeiten von erfindungsgemäß geeigneten ringförmigen Vollkatalysatoren 5 bis 15 N, häufig 8 bis 11 N. Günstig sind erfindungsgemäß zu verwendende ringförmige Vollkatalysatoren vor allem dann, wenn die vorstehenden Seitendruckfestigkeiten und gleichzeitig die vorstehenden Kombinationen von O, V, Porenverteilung A oder Porenverteilung B sowie gegebenenfalls d^{max} vorliegen.

Das erfindungsgemäß Verfahren der partiellen, heterogen katalysierten, Gasphasenoxidation von Propen zu Acrolein kann im übrigen wie in den Schriften WO 00/53557, WO 00/53558, DE-A 19910506, EP-A 1106598, WO 01/36364, DE-A 19927624, WO 00/53557, DE-A 19948248, DE-A 19948523, DE-A 19948241, EP-A 700714, DE-A 10313213, DE-A 10313209, DE-A 10232748, DE-A 10313208, WO 03/038744, EP-A 279374, DE-A 3338380, DE-A 3300044, EP-A 575897, DE-A 4407020, DE-A 10344149, DE-A 10351269 und DE-A 10350812 beschrieben durchgeführt werden, wobei das Katalysatorfestbett z.B. nur erfindungemäß zu verwendende ringförmige Vollkatalysatoren oder mit inerten Formkörpern verdünnte ringförmige Vollkatalysatoren umfassen kann. Im letzteren Fall wird das Katalysatorfestbett erfindungsgemäß vorteilhaft in der Regel so gestaltet, dass ihre volumenspezifische Aktivität in Strömungsrichtung des Reaktionsgasgemischs kontinuierlich, sprunghaft und/oder stufenförmig zunimmt.

Die Vorteilhaftigkeit der beim erfindungsgemäßen Verfahren zu verwendenden ringförmigen Vollkatalysatoren zeigt sich vor allem dann, wenn die Belastung des Katalysatorfestbetts mit Propen ≥ 130 NI/I•h, oder ≥ 140 NI/I•h, oder ≥ 150 NI/I•h, oder ≥ 160 NI/I•h beträgt. Im Normalfall wird die vorgenannte Belastung des Katalysatorfestbetts ≤ 600 NI/I•h, häufig ≤ 500 NI/I•h, vielfach ≤ 400 Nl/1•h oder ≤ 350 NI/I•h betragen. Propenbelastungen des Katalysatorfestbetts im Bereich von 160 NI/I•h bis 300 bzw. 250 oder 200 NI/I•h sind besonders typisch.

Selbstverständlich kann das erfindungsgemäße Verfahren auch bei nicht erfindungsgemäßen Propenbelastungen des zu verwendenden Katalysatorfestbetts von < 120 NI/I•h, oder ≤ 110 NI/I•h, oder ≤ 100 NI/I•h betrieben werden. In der Regel werden solche nicht erfindungsgemäßen Propenbelastungen jedoch bei Werten ≥ 60 NI/I•h, oder ≥ 80 NI/I•h liegen.

Unter der "Propenbelastung" des Katalysatorfestbetts wird in dieser Schrift (wie allgemein üblich) die Menge an Propen in Normlitern (= NI; das Volumen in Liter, das die entsprechende Propenmenge bei Normalbedingungen, d.h., bei 25°C und 1 bar, einnehmen würde) verstanden, die als Bestandteil des Reaktionsgasausgangsgemischs pro Stunde durch einen Liter an Katalysatorfestbett geführt wird. Vor- und/oder Nachschüttungen aus reinem Inertmaterial werden dabei nicht als zum Katalysatorfestbett zugehörig betrachtet. Belastungen des Katalysatorfestbetts mit Reaktionsgasausgangsgemisch werden entsprechend verstanden, lediglich "Propen" ist dabei durch "Reaktionsgasausgangsgemisch" zu ersetzen.

Prinzipiell kann die Belastung des Katalysatorfestbetts mit Propen beim erfindungsgemäßen Verfahren über zwei Stellschrauben eingestellt werden:
a) die Belastung des Katalysatorfestbetts mit Reaktionsgasausgangsgemisch und/oder
b) den Gehalt des Reaktionsgasausgangsgemischs mit Propen.

Das erfindungsgemäße Verfahren eignet sich sowohl dann, wenn bei ≥ 120 NI/I•h liegenden Propenbelastungen des Katalysatorfestbetts die Belastungseinstellung über die vorgenannte Stellschraube a) erfolgt, als auch wenn die Belastungseinstellung über die vorgenannte Stellschraube b) erfolgt. In der Regel erweist sich eine Hochlasteinstellung über die Stellschraube a) als vorteilhaft, da der damit verbundene vergleichsweise geringere Propenpartialdruck mit einer geringeren Verweilzeit im Katalysatorgefüge einhergeht und gleichzeitig eine Blockierung der aktiven Zentren an der Katalysatoroberfläche erschwert. Außerdem geht mit einer solchen Verfahrensweise ein vorteilhafterer radialer und axialer Wärmeabtransport einher.

Der Propenanteil im Reaktionsgasausgangsgemisch wird beim erfindungsgemäßen Verfahren im Regelfall (d.h. im wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen). Erfindungsgemäß günstige Propenanteile sind auch die entsprechend bezogenen Bereiche 7 bis 15 Vol.-% bzw. 8 bis 12 Vol.-%, oder 9 bis 11 Vol.-%, sowie 5 bis 8 Vol.-%.

Häufig wird man das Verfahren der mit den erfindungsgemäß erhältlichen ringförmigen Vollkatalysatoren katalysierten Partialoxidation (im wesentlichen unabhängig von der Belastung) bei einem Propen: Sauerstoff : indifferente Gase (einschließlich Wasserdampf) Volumenverhältnis im Reaktionsgasausgangsgemisch von 1 :(1 ,0 bis 3,0):(3 bis 30), vorzugsweise 1 :(1,5 bis 2,3):(10 bis 15) durchführen.

D.h., in der Regel wird im Reaktionsgasausgangsgemisch das molare Verhältnis O₂: C₃H₆ ≤ 3 betragen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu ≥ 20 Vol.-%, oder zu ≥ 30 Vol.-%, oder zu ≥ 40 Vol.-%, oder zu ≥ 50 Vol.- %, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Das indifferente Verdünnungsgas kann aber auch z.B. zu 2 bis 35 bzw. 20 Vol.-% aus H₂O und zu 65 bis 98 Vol.-% aus N₂ bestehen.

Bei Belastungen des Katalysatorfestbetts mit Propen von ≥ 250 NI/I•h ist jedoch die Mitverwendung von inerten Verdünnungsgasen wie Propan, Ethan, Methan, Pentan, Butan, CO₂, CO, Wasserdampf und/oder Edelgasen für das Rektionsgasausgangsgemisch empfehlenswert. Generell können diese inerten Gase und ihre Gemische aber auch bereits bei geringeren erfindungsgemäßen Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im wesentlichen selektiv abtrennt. Dabei ist zu berücksichtigen, dass die erfindungsgemäße Partialoxidation zu Acrolein mit den erfindungsgemäß zu verwendenden ringförmigen Vollkatalysatoren nur die erste Stufe einer zweistufigen Partialoxidation des Propens zu Acrylsäure als der eigentlichen Zielverbindung sein kann, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Erfindungsgemäß wesentlich ist dabei lediglich, dass das Reaktionsgasausgangsgemisch Kohlendioxid und gesättigte Kohlenwasserstoffe zusammen in einer Gesamtmenge von ≤ 15 mol-% (bzw. 15 Vol.-%), vorzugsweise von ≤ 10 mol-% und besonders bevorzugt ≤ 5 mol-% bzw. ≤ 3 mol-% enthält.

Bei der erfindungsgemäßen Partialoxidation von Propen zu Acrolein, unter Anwendung der erfindungsgemäß zu verwendenden ringförmigen Vollkatalysatoren, kann eine typische Zusammensetzung des Reaktionsgasausgangsgemischs (unabhängig von der gewählten Belastung) z.B. die nachfolgenden Komponenten enthalten:
6 bis 6,5 Vol.% Propen,
3 bis 3,5 Vol.-% H₂O,
0,3 bis 0,5 Vol.-% CO,
0,8 bis 1,2 Vol.-% CO₂,
0,025 bis 0,04 Vol.-% Acrolein,
10,4 bis 10,7 Vol.-% O₂ und
als Restmenge ad 100% (im wesentlichen) molekularer Stickstoff,

oder: 5,4 Vol.-% Propen,
10,5 Vol.-% Sauerstoff,
1,2 Vol.-% COx,
81,3 Vol.-% N₂ und
1,6 Vol.-% H₂O.

Das Reaktionsgasausgangsgemisch kann aber auch wie folgt zusammengesetzt sein:
7 bis 15 Vol.-% Propen,
4 bis 30 Vol.-% (häufig 6 bis 15 Vol.-%) Wasser,
≥ 0 bis 10 Vol.-% (vorzugsweise ≥ 0 bis 5 Vol.-%) von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus (im wesentlichen ) molekularem Stickstoff.

Eine andere mögliche Reaktionsgasausgangsgemischzusammensetzung kann enthalten:
6,0 Vol.-% Propen,
60 Vol.-% Luft und
34 Vol.-% H₂O.

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Weitere erfindungsgemäß geeignete Reaktionsgasausgangsgemische können im nachfolgenden Zusammensetzungsraster liegen:
7 bis 11 Vol.-% Propen,
6 bis 12 Vol.-% Wasser,
≥ 0 bis 5 Vol.-% von Propen, Wasser, Sauerstoff und Stickstoff verschiedenen Bestandteilen,
soviel molekularem Sauerstoff, dass das molare Verhältnis von enthaltenem Sauerstoff zu enthaltenem molekularem Propen 1,6 bis 2,2 beträgt, und
als Restmenge bis zur 100 Vol.-% Gesamtmenge (im wesentlichen) molekularer Stickstoff.

Die Reaktionstemperatur für die Propenpartialoxidation (d.h., die Temperatur des Katalysatorfestbetts) liegt bei Verwendung der erfindungsgemäß anzuwendenden ringförmigen Vollkatalysatoren häufig bei 150 bis 450°C, vorzugsweise im Bereich von 300 bis 400°C und besonders bevorzugt bei 300 bis 380°C.

Der Reaktionsdruck liegt für die vorgenannte Partialoxidation in der Regel bei 0,5 bzw. 1,5 bis 3 bzw. 4 bar.

Die Gesamtbelastung des Katalysatorfestbetts mit Reaktionsgasausgangsgemisch beläuft sich bei der erfindungsgemäßen Partialoxidationen typisch auf 1000 bis 10000 NI/I-h, meist auf 1500 bis 5000 Ni/t-h und oft auf 2000 bis 4000 NWh.

Als im Reaktionsgasausgangsgemisch zu verwendendes Propen kommen vor allem polymer grade Propen und chemical grade Propen in Betracht, wie es z.B. die DE-A 10232748 beschreibt.

Als Sauerstoffquelle wird normalerweise Luft eingesetzt.

Die Durchführung der erfindungsgemäßen Partialoxidation in Anwendung der erfindungsgemäß zu verwendenden ringförmigen Vollkatalysatoren kann im einfachsten Fall z.B. in einem Einzonen-Vielkontaktrohr-Festbettreaktor erfolgen, wie ihn die DE-A 44 31 957, die EP-A 700 714, die WO 03/057653, die WO 03/055835, die WO 03/059857, die WO 03/076373 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Eine typische Kontaktrohrlänge beläuft sich z.B. auf 3,20 m. Anwendungstechnisch zweckmäßig beläuft sich die im Rohrbündelbehälter untergebrachte Anzahl an Kontaktrohren auf wenigstens 5000, vorzugsweise auf wenigstens 1000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Durchführung der erfindungsgemäßen Partialoxidation kann aber auch in einem Mehrzonen (z.B. "Zwei") -Vielkontaktrohr-Festbettreaktor durchgeführt werden, wie es die DE-A 19948523, die DE-A 199 10 506, die DE-A 10313213, die DE-A 10313208 und die EP-A 1 106 598 insbesondere bei besonders hohen Belastungen des Katalysatorfestbetts mit Propen empfehlen (z.B. bei ≥ 160 NI/I•h). Eine typische Kontaktrohrlänge im Fall eines Zweizonen-Vielkontaktrohr-Festbettreaktors beträgt 3,50 m. Alles andere gilt im wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z.B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittstelle aus der Temperaturzone um 0 bis 15°C, häufig 1 bis 10°C, oder 2 bis 8°C, oder 3 bis 6°C ansteigt.

Die Eingangstemperatur des Wärmeaustauschmittels, das, über die jeweilige Temperierzone (einschließlich der Einzone beim Einzonen-Vielkontaktrohr-Festbettreaktor) betrachtet, im Gleichstrom oder im Gegenstrom zum Reaktionsgasgemisch geführt werden kann (wobei die Gleichstromfahrweise erfindungsgemäß besonders bevorzugt ist), wird vorzugsweise wie in den Schriften EP-A 1 106 598, DE-A 19948523, DE-A 19948248, DE-A 10313209, EP-A 700 714, DE-A 10313208, DE-A 10313213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohr und Arbeitsrohren gleich ist (vgl. EP-A 873783, WO 03-076373 und EP-A 1270065).

Der Druckverlust sollte bei Arbeitsrohren und Thermorohr, bezogen auf gleiche GHSV, gleich sein. Ein Druckverlustangleich beim Thermorohr kann durch Zusatz von versplitteten Katalysatorringen zu den ringförmigen Vollkatalysatoren erfolgen. Dieser Ausgleich erfolgt zweckmäßig über die gesamte Thermorohrlänge homogen.

Zur Bereitung des Katalysatorfestbetts in den Kontaktrohren können beim erfindungsgemäßen Verfahren, wie bereits erwähnt, nur erfindungsgemäß zu verwendende ringförmige Vollkatalysatoren oder z.B. auch weitgehend homogene Gemische aus erfindungsgemäß zu verwendenden ringförmigen Vollkatalysatoren und keine Aktivmasse aufweisenden, sich bezüglich der heterogen katalysierten partiellen Gasphasenoxidation von Propen zu Acrolein im wesentlichen inert verhaltenden Formkörpern verwendet werden. Als Materialien für solche inerten Formkörper kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie die Katalysatorformkörper, Ringe sein. Häufig wird man als inerte Verdünnungsformkörper solche wählen, deren Geometrie derjenigen der mit ihnen zu verdünnenden ringförmigen Vollkatalysatoren entspricht. Längs des Katalysatorfestbetts kann aber auch die Ringgeometrie gewechselt werden. In einer weniger bevorzugten Vorgehensweise kann auch die Aktivmasse der ringförmigen Vollkatalysatoren längs des Katalysatorfestbetts verändert werden, so lange sie Bestandteil der allgemeinen Formel 1 bleibt.

Ganz generell wird, wie bereits erwähnt, das Katalysatorfestbett mit Vorteil so gestaltet, dass die volumenspezifische (d.h., die auf die Einheit des Volumens normierte) Aktivität in Strömungsrichtung des Reaktionsgasgemisches entweder konstant bleibt oder zunimmt (kontinuierlich, sprunghaft oder stufenförmig).

Eine Verringerung der volumenspezifischen Aktivität kann in einfacher Weise z.B. dadurch erzielt werden, dass man eine Grundmenge von erfindungsgemäß einheitlich hergestellten ringförmigen Vollkatalysatoren mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß zu verwendenden ringförmigen Vollkatalysatoren so verändert, dass die in der Einheit des Ringgesamtvolumens (einschließlich der Ringöffnung) enthaltene Aktivmassenmenge kleiner wird.

Für die erfindungsgemäße heterogen katalysierte Gasphasenpartialoxidation mit den erfindungsgemäß zu verwendenden ringförmigen Vollkatalysatoren wird das Katalysatorfestbett vorzugsweise entweder auf der gesamten Länge einheitlich mit nur einem Vollkatalysatorring gestaltet oder wie folgt strukturiert. Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Katalysatorfestbetts, ein im wesentlichen homogenes Gemisch aus Verdünnungsformkörper und erfindungsgemäß zu verwendende ringförmigem Vollkatalysator (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge des Katalysatorfestbetts (d.h., z.B. auf einer Länge von 2,00 bis 3,00 m, bevorzugt 2,50 bis 3,00 m) entweder eine nur in geringerem Umfang (als im ersten Abschnitt) verdünnte Schüttung des erfindungsgemäß zu verwendenden ringförmigen Vollkatalysators, oder, ganz besonders bevorzugt, eine alleinige (unverdünnte) Schüttung desselben ringförmigen Vollkatalysators, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß zu verwendenden ringförmigen Vollkatalysator von geringer, auf seinen Raumbedarf bezogener, Aktivmassendichte und im zweiten Abschnitt mit einem erfindungsgemäß zu verwendenden ringförmigen Vollkatalysator mit hoher, auf seinen Raumbedarf bezogener, Aktivmassendichte beschickt werden (z.B. 6,5 mm x 3 mm x 4,5 mm [A x L x I] im ersten Abschnitt, und 5 x 2 x 2 mm im zweiten Abschnitt).

Insgesamt werden bei einer erfindungsgemäßen Partialoxidation von Propen zu Acrolein das Katalysatorfestbett, das Reaktionsgasausgangsgemisch, die Belastung und die Reaktionstemperatur (Temperatur des Katalysatorfestbetts) in der Regel so gewählt, dass beim einmaligen Durchgang des Reaktionsgasgemischs durch das Katalysatorfestbett ein Umsatz des partiell zu oxidierenden Propen von wenigstens 90 mol-%, oder wenigstens 92 mol-%, vorzugsweise von wenigstens 95 mol-% oder wenigstens 97 mol-% resultiert. Die Selektivität der Acroleinbildung wird dabei regelmäßig ≥ 80 mol-%, bzw. ≥ 82 mol-%, bzw. ≥ 84 mol-%, bzw. ≥ 85 mol-%, oder ≥ 86 mol-%, oder ≥ 87 mol-% betragen. In natürlicher Weise resultieren dabei besonders geringe Heißpunkttemperaturen. Gleichzeitig resultiert dabei eine erhöhte Selektivität der Zielproduktbildung.

Abschließend sei festgehalten, das die erfindungsgemäß zu verwendenden ringförmigen Vollkatalysatoren auch ein vorteilhaftes Bruchverhalten bei der Reaktorbefüllung aufweisen. Auch ihr Druckverlustverhalten ist vorteilhaft. Im übrigen eignen sich die erfindungsgemäß zu verwendenden ringförmigen Vollkatalysatoren ganz generell als Katalysatoren mit erhöhter Aktivität und Selektivität für gasphasenkatalytische Partialoxidationen organischer Verbindungen wie niederer (z.B. 3 bis 6 (d.h. 3, 4, 5, oder 6) C-Atome enthaltender) Alkane, Alkanole, Alkanale, Alkene und Alkenale zu olefinisch ungesättigten Adehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytisch oxidative Dehydrierungen organischer Verbindungen (z.B. 3, 4, 5, oder 6 C-Atome enthaltender). Im Fall von Methacrolein als Zielverbindung kann dabei wie in der DE-A 4407020 verfahren werden.

Der Wismutgehalt der erfindungsgemäß erhältlichen Aktivmassen kann auch wie in der DE-A 100 63 162 beschrieben eingestellt werden. Dabei wird aus Ausgangsverbindungen der elementaren Konstituenten der angestrebten Aktivmasse eine Lösung oder Suspension erzeugt, die zwar die zur Herstellung der Aktivmasse erforderliche Gesamtmenge der von Bi verschiedenen elementaren Konstituenten, aber nur eine Teilmenge des zur Herstellung der Aktivmasse erforderlichen Bi enthält, die Lösung oder Suspension unter Erhalt einer Trockenmasse getrocknet und die zur Herstellung der Aktivmasse zusätzlich benötigte Restmenge an Bi in Form einer Ausgangsverbindung des Bi wie in der DE-A 100 63 162 beschrieben unter Erhalt eines formbaren Gemischs in diese Trockenmasse eingearbeitet (z.B. wie im Beispiel der DE-A 100 63 162), das formbare Gemisch in erfindungsgemäßer Weise (gegebenenfalls nach Zugabe von Formungs- und/oder Verstärkungshilfsmitteln) zu einem ringförmigen Vollkatalysatorformkörper geformt und dieser durch thermisches Behandeln (z.B. wie im Beispiel der DE-A 100 63 162) in den gewünschten ringförmigen Vollkatalysator überführt.

Ein Vorteil der erfindungsgemäßen Verfahrensweise besteht auch darin, dass bei infolge variierender Marktnachfrage nach Zielprodukt erforderlicher Änderung der Propenlast des Katalysatorfestbetts solche Anpassungen möglich sind, ohne dass damit bezogen auf gleichbleibenden Propenumsatz bei einmaligem Durchgang des Reaktionsgasgemischs, ausgeprägte Änderungen der Temperatur des Katalysatorfestbetts einhergehen müssen. Dies erleichtert die Anlagenauslegung erheblich.

Die Inbetriebnahme eines frischen Katalysatorfestbetts (bzw. eines frisch regenerierten Katalysatorfestbetts) mit erfindungsgemäß zu verwendenden ringförmigen Vollkatalysatoren kann wie in der DE-A 10337788 beschrieben erfolgen. In der Regel nehmen Aktivität und Selektivität der Zielproduktbildung anfänglich mit der Betriebsdauer der Katalysatorbeschickung zu. Diese Formierung kann dadurch beschleunigt werden, dass man sie bei im wesentlichen gleichbleibendem Umsatz unter erhöhter Belastung der Katalysatorbeschickung mit Reaktionsgasausgangsgemisch durchführt und nach weitgehend vollzogener Formierung die Belastung auf ihren Zielwert zurücknimmt.

Die Regenerierung eines erfindungsgemäß betriebenen Katalysatorfestbetts kann wie in den Schriften DE-A 10351269, DE-A 10350812, EP-A 169449, EP-A 614872 und EP-A 339119 beschrieben erfolgen.

Abschließend sei noch festgehalten, dass das erfindungsgemäße Verfahren in nicht erfindungsgemäßer Weise auch mit Vollkatalysatoren aus Multimetalloxiden der allgemeinen Formel I aber mit einer von einer ringförmigen Geometrie verschiedenen Geometrie gemäß der DE-A 10101695 durchgeführt werden kann, wobei die Propenbelastung ≥ 120 NI/I•h oder < 120 NI/I•h betragen kann.

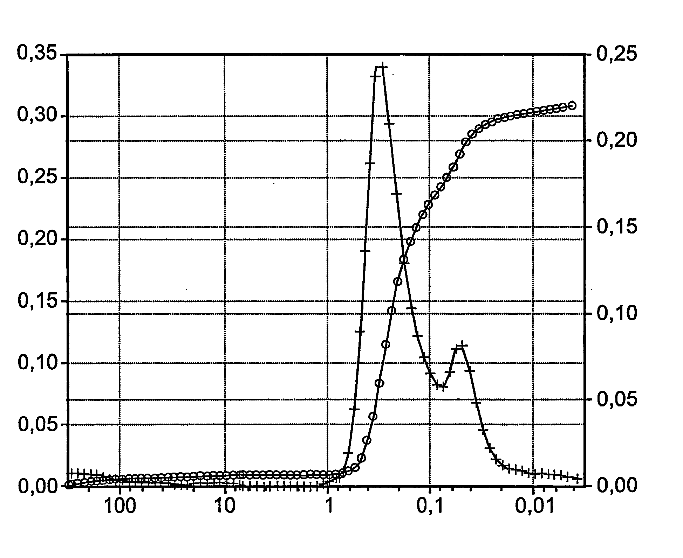

Besonders bevorzugte Vollkatalysatorgeometrien sind dabei vor allem jene gemäß Fig. 1A sowie Fig. 2A der DE-A 10101695.

### Beispiel und Vergleichsbeispiel

### A) Herstellung eines ringförmigen Vergleichsvollkatalysators WK mit der nachfolgenden Stöchiometrie VS der Aktivmasse: Mo₁₂W₂Co_{5,5}Fe₃Bi₁Si_{1,6}K_{0,08}Oₓ

### 1. Herstellung einer Ausgangsmasse 1

In 775 kg einer wässrigen salpetersauren Wismutnitratlösung (11,2 Gew.-% Bi; freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wässrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver (Partikelgröße im wesentlichen einheitlich 30 µm), das einen Glühverlust von 12 Gew.-% aufwies (3 h bei 600°C unter Luft glühen), wurde anschließend mit 16,8 Gew.-% (bezogen auf das Pulver) Wasser in einem Kneter angeteigt und mittels eines Exruders (Drehmoment: ≤ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min bei Temperaturen von 90-95°C (Zone 1), 115°C (Zone 2) und 125°C (Zone 3) an Luft getrocknet und dann bei einer Temperatur im Bereich von 780 bis 810°C thermisch behandelt (calciniert; im luftdurchströmten Drehrohrofen (0,3 mbar Unterdruck, 1,54 m³ Innenvolumen, 200 Nm³ Luft/h)). Wesentlich bei der genauen Einstellung der Calcinationstemperatur ist, dass sie an der angestrebten Phasenzusammensetzung des Calcinationsprodukts orientiert zu erfolgen hat. Gewünscht sind die Phasen WO₃ (monoklin) und Bi₂W₂O₉, unerwünscht ist das Vorhandensein von γ-Bi₂WO₆ (Russellit). Sollte daher nach der Calcination die Verbindung γ-Bi₂WO₆ anhand eines Reflexes im Pulverröntgendiffraktogramm bei einem Reflexwinkel von 20 = 28,4° (CuKα-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde gemahlen, so dass der X₅₀-Wert (vgl. Ullmann's Encyclopedia of Industrial Chemistry, 6th Edition (1998) Electronic Release, Kapitel 3.1.4 oder DIN 66141) der resultierenden Körnung 5 mm betrug. Das Mahlgut wurde dann mit 1 Gew.-% (bezogen auf das Mahlgut) feinteiligem SiO₂ der Fa. Degussa vom Typ Sipernat^{®} (Rüttelgewicht 150 g/l; X₅₀-Wert der SiO₂-partikel betrug 10 µm, die BET-Oberfläche betrug 100 m²/g) vermischt.

### 2. Herstellung einer Ausgangsmasse 2

Eine Lösung A wurde hergestellt, indem man bei 60°C unter Rühren in 600 I Wasser 213 kg Ammoniumheptamolybdattetrahydrat (81,5 Gew.-% MoO₃) löste und die resultierende Lösung unter Aufrechterhaltung der 60°C und Rühren mit 0,97 kg einer 20°C aufweisenden wässrigen Kaliumhydroxidlösung (46,8 Gew.-% KOH) versetzte.

Eine Lösung B wurde hergestellt indem man bei 60°C in 262,9 kg einer wässrigen Co-(II)-baltnitratlösung (12,4 Gew.-% Co) 116,25 kg einer wässrigen Eisen-(III)-nitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wässrigen Gemisch 19,16 kg eines Kieselgels der Fa. Dupont vom Typ Ludox (46,80 Gew.-% SiO₂, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400 ± 10°C, Gasaustrittstemperatur: 140 ± 5°C). Das resultierende Sprühpulver wies einen Glühverlust von ca. 30 Gew.-% (3 h bei 600°C unter Luft glühen) und eine im wesentlichen einheitliche Körnung von 30 µm auf.

### 3. Herstellung der Multimetalloxidaktivmasse

Die Ausgangsmasse 1 wurde mit der Ausgangsmasse 2 in den für eine Multimetalloxidaktivmasse der Vergleichsstöchiometrie

Mo₁₂W₂Co_{5,5}Fe₃Bi₁Si_{1,6}K_{0,08}Oₓ

erforderlichen Mengen in einem Mischer mit Messerköpfen homogen vermischt. Bezogen auf die vorgenannte Gesamtmasse wurden zusätzlich 1 Gew.-% feinteiliges Graphit der Fa. Timcal AG (San Antonio, US), vom Typ TIMREX P44 (Siebanalyse: min. 50 Gew.-% < 24 mm, max. 10 Gew.-% ≥ 24 µm und ≤ 48 µm, max. 5 Gew.-% > 48 µm, BET-Oberfläche: 6 bis 13 m²/g) homogen eingemischt. Das resultierende Gemisch wurden dann in einem Kompaktor (Fa. Hosokawa Bepex GmbH, D-74211 Leingarten) vom Typ Kompaktor K200/100 mit konkaven, geriffelten Glattwalzen gefahren (Spaltweite: 2,8 mm, Siebweite: 1,0 mm, Siebweite Unterkorn: 400 µm, Presssollkraft: 60 kN, Schneckendrehzahl: 65 bis 70 Umdrehungen je Minute). Das resultierende Kompaktat wies eine Härte von 10N und eine im wesentlichen einheitliche Körnung von 400 µm bis 1 mm auf.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ Rx 73, der Fa. Kilian, D-50735 Köln, unter Stickstoffatmosphäre zu einem ringförmigen Vollkatalysatorvorläuferformkörper der Geometrie (A x L x I) 6 mm x 3 mm x 4 mm mit einer Seitendruckfestigkeit von 16,3 N verdichtet.

Zur abschließenden thermischen Behandlung wurden 1000 g der Vollkatalysatorvorläuferformkörper in einem Luft durchströmten Muffelofen (60 I Innenvolumen, 1 I/h Luft pro Gramm Vollkatalysatorvorläuferformkörper) zunächst mit einer Aufheizrate von 180°C/h von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten, bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.

Dabei wurden aus dem ringförmigen Vollkatalysatorvorläuferformkörper der nachfolgende ringförmige Vergleichsvollkatalysator erhalten:

| | |
|---|---|
| Spezifische Oberfläche O: | 9,61 cm²/g. |
| Porengesamtvolumen V: | 0,22 cm³/g. |
| d^{max} [µm]: | 0,30. |
| R: | 0,68. |

Prozentualer Anteil derjenigen Porendurchmesser am Porengesamtvolumen, deren Durchmesser > 0,1 und < 1 µm betragen: V₁^{0,1}-% = 70.

Die Figur 1 zeigt außerdem die Porenverteilung des ringförmigen Vergleichsvollkatalysators VS. Auf der Abszisse ist der Porendurchmesser in µm aufgetragen (logarithmische Skala). Auf der linken Ordinate ist der Logarithmus des differentiellen Beitrags in ml/g des jeweiligen Porendurchmessers zum Porengesamtvolumen aufgetragen (Kurve +). Das Maximum weist den Porendurchmesser mit dem größten Beitrag zum Porengesamtvolumen aus. Auf der rechten Ordinate ist in ml/g das Integral über die individuellen Beiträge der einzelnen Porendurchmesser zum Porengesamtvolumen aufgetragen (Kurve O). Der Endpunkt ist das Porengesamtvolumen.

### B) Herstellung eines erfindungsgemäßen ringförmigen Vollkatalysators BVK mit der nachfolgenden erfindungsgemäßen Stöchiometrie BS der Aktivmasse: Mo₁₂W₂Co₇Fe₃Bi₁Si_{1,6}K_{0,08}O_{y}

Die Herstellung erfolgte wie beim Vergleichsvollkatalysator WK, jedoch mit dem Unterschied, dass bei der Herstellung der Lösung B die Menge der wässrigen Co-(II)baltnitratlösung von 262,9 kg auf 334,6 kg erhöht wurde. Ferner wurde die Ausgangsmasse 1 mit der Ausgangsmasse 2 in den für eine Multimetalloxidaktivmasse der erfindungsgemäßen Stöchiometrie Mo₁₂W₂Co₇Fe₃Bi₁Si_{1,6}K_{0,08}O_{y} erforderlichen Mengen vermischt.

Die physikalischen Eigenschaften der resultierenden erfindungsgemäßen ringförmigen (6 mm x 3 mm x 4 mm) Vollkatalysators BVK (O, V, d^{max}, R, V₁^{0,1}-%) waren von jenen des ringförmigen Vergleichsvollkatalysators VVK innerhalb der Reproduzierbarkeit nicht unterscheidbar.

Anstatt die thermische Behandlung wie bei der Herstellung des Vergleichsvollkatalysators VVK beschrieben durchzuführen, kann man sie sowohl bei der Herstellung von WK als auch von BVK auch wie in Beispiel 1 der DE-A 10046957 (die Schütthöhe in der Zersetzung (Kammern 1 bis 4) beläuft sich dabei jedoch vorteilhaft auf 44 mm bei einer Verweilzeit pro Kammer von 1,46 h und in der Calcination (Kammern 5 bis 8) beläuft sie sich vorteilhaft auf 130 mm bei einer Verweilzeit von 4,67 h) beschrieben mittels einer Bandcalziniervorrichtung durchführen; die Kammern besitzen eine Grundfläche (bei einer einheitlichen Kammerlänge von 1,40 m) von 1,29 m² (Zersetzung) und 1,40 m² (Calcination) und werden von unten durch das grobmaschige Band von 75 Nm³/h Zuluft durchströmt, die mittels rotierender Ventilatoren angesaugt werden. Innerhalb der Kammern ist die zeitliche und örtliche Abweichung der Temperatur vom Sollwert stets ≤ 2°C. Im übrigen wird wie in Beispiel 1 der DE-A 10046957 beschrieben verfahren. Die resultierenden ringförmigen Vollkatalysatoren können wie die ringförmigen Vollkatalysatoren WK und BVK für die nachfolgend unter C) beschriebenen gasphasenkatalytischen Partialoxidationen von Propen zu Acrolein eingesetzt werden.

Als weitere Alternative kann die thermische Behandlung in einem Umluftofen (z.B. in einem solchen der Fa. Elino vom Typ Laborkammerofen KA-040/006-08 EW.OH bzw. einem solchen der Fa. Heraeus vom Typ K 750) so durchgeführt werden, dass man innerhalb von 6 h auf 270°C erwärmt und anschließend so lange die 270°C aufrechterhält, bis die Umluft frei ist von nitrosen Gasen. Nachfolgend wird innerhalb von 1,5 h auf eine Temperatur von 430°C bis 460°C (vorzugsweise auf 438°C) erwärmt und diese Temperatur 10 h aufrechterhalten. Die Luftspülung beträgt 800 NI/h. 1000 g ringförmige Vollkatalysatorvorläuferformkörper sind in einen quaderförmigen Drahtkorb (10 cm hoch, 14 cm x 14 cm Grundfläche) in einer Schütthöhe von ca. 4 cm eingefüllt. Die Restgrundfläche des Tragekorbes wird in entsprechender Schütthöhe mit Steatitringen (wie immer in den Beispielen und Vergleichsbeispielen vom Typ C220 der Fa. Ceram Tec, DE) gleicher Geometrie belegt.

Die resultierenden ringförmigen Vollkatalysatoren können wie die ringförmigen Vollkatalysatoren VVK und BVK in den unter C) beispielhaft beschriebenen gasphasenkatalytischen Partialoxidationen eingesetzt werden.

### C) Testung der in A) und B) hergestellten ringförmigen Vollkatalysatoren VVK bzw. BVK für eine heterogen katalysierte Partialoxidation von Propen zu Acrolein

### 1. Versuchsanordnung

Ein Reaktionsrohr (V2A Stahl; 30 mm Außendurchmesser, 2 mm Wandstärke, 26 mm Innendurchmesser, 320 cm Länge, ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann.

In Strömungsrichtung wurde dabei wie folgt beschickt:

| | |
|---|---|
| Abschnitt 1: | 50 cm Länge Steatitringe der Geometrie 7 mm x 7 mm x 4 mm (Außendurchmesser x Länger x Innendurchmesser) als Vorschüttung. |
| | |
| Abschnitt 2: | 270 cm Länge Katalysatorbeschickung mit den ringförmigen Vollkatalysatoren VVK bzw. BVK. |

Die Temperierung des Reaktionsrohres erfolgte mittels eines im Gegenstrom gepumpten Salzbades.

### 2. Versuchsdurchführung

Die beschriebene, jeweils frisch hergerichtete, Versuchsanordnung wurde jeweils kontinuierlich mit einem Beschickungsgasgemsich (Reaktionsgasausgangsgemisch) der Zusammensetzung
5,4 Vol.-% Propen (chemical grade Propylen),
10,5 Vol.-% Sauerstoff,
1,2 Vol.-% COₓ,
81,3 Vol.-% N₂ und
1,6 Vol.-% H₂O
beschickt, wobei die Thermostatisierung des Reaktionsrohres bei gegebener Belastung PL (NI/I•h) des Katalysatorfestbetts mit im Reaktionsgasausgangsgemisch enthaltenem Propen so erfolgte, dass der Propenumsatz U (mol-%) bei einmaligem Durchgang des Reaktionsgasausgangsgemischs durch das Reaktionsrohr kontinuierlich etwa 95 mol-% betrug.

Die nachfolgende Tabelle zeigt die in Abhängigkeit vom gewählten Katalysatorfestbett bei der gewählten Propenbelastung von 150 NI/I•h sich einstellende Heißpunkttemperatur (T^{HS} (°C)) sowie die erzielte Selektivität S^{A} der Acroleinbildung in mol-% und bezogen auf im einfachen Durchgang umgesetztes Propen.

Die angegebenen Ergebnisse beziehen sich stets auf das Ende einer Betriebsdauer von 120 h.

Ferner zeigt die Tabelle um wie viel °C die Salzbadtemperatur abgesenkt werden musste (ΔT^{S}), um bei einer (unter ansonsten gleichbleibenden Bedingungen) Absenkung von PL von 150 NI/I•h auf 100 NI/I•h den Propenumsatz aufrechtzuerhalten.

**Tabelle**

| | T^{HS} (°C) | S^{A} (mol-%) | ΔT^{S} (°C) |
|---|---|---|---|
| WK | 424 | 87,9 | 27 |
| BVK | 392 | 88,3 | 7 |

Die Ergebnisse weisen aus, dass alle Vorteile auf Seiten des Katalysatorfestbetts BVK liegen. Die Selektivität der Acrylsäurenebenproduktbildung lag in allen Fällen bei etwa 8,5 mol-% (bezogen auf umgesetztes Propen).

Die vorbeschriebene Versuchsdurchführung kann in entsprechender Weise auch mit einer Katalysatorbeschickung durchgeführt werden, deren Abschnitt 2 wie folgt gestaltet ist (jeweils in Strömungsrichtung):
I. Zunächst auf 100 cm Länge ein homogenes Gemisch aus 65 Gew.-% BVK und 35 Gew.-% Steatitringen (5 mm x 3 mm x 2 mm); anschließend auf 170 cm Länge ein homogenes Gemisch aus 90 Gew.-% BVK und 10 Gew.-% Steatitringen (5 mm x 3 mm x 2 mm);
   oder
II. Zunächst auf 100 cm Länge ein homogenes Gemisch aus 70 Gew.-% BVK und 30 Gew.-% Steatitringen (5 mm x 3 mm x 2 mm);
anschließend auf 170 cm Länge BVK.

Die Salzbadtemperatur wird stets so gewählt, das U-Propen = 95 mol-% bei einmaligem Durchgang.

Alle beschriebenen Beispiele und Vergleichsbeispiele können auch mit einem Reaktionsgasausgangsgemisch durchgeführt werden, das anstatt 5,4 Vol.-% Propen, 6,7 Vol.-% Propen und anstatt 81,3 Vol.-% N₂, 80 Vol.-% N₂ enthält.

Auch kann der ringförmige Katalysator BVK in den Ausführungs- und Vergleichsbeispielen der Schriften DE-A 10313210, DE-A 10313213, DE-A 10313212 und DE-A 10313208 den in diesen Schriften verwendeten ringförmigen Vollkatalysator ersetzen.

Im Hinblick auf die obengenannten Lehren sind zahlreiche Änderungen und Abweichungen von der vorliegenden Erfindung möglich. Man kann deshalb davon ausgehen, dass die Erfindung im Rahmen der beigefügten Ansprüche, anders als hierin spezifisch beschrieben, ausgeführt werden kann.

## Patentansprüche

1. Verfahren zur Herstellung von Acrolein durch heterogen katalysierte partielle Gasphasenoxidation, bei dem man ein Propen, molekularen Sauerstoff und wenigstens ein Inertgas enthaltendes Reaktionsgasausgangsgemisch, das den molekularen Sauerstoff und das Propen in einem molaren Verhältnis O₂ : C₃H₆ ≥1 sowie Kohlendioxid und gesättigte Kohlenwasserstoffe zusammen in einer Gesamtmenge von höchstens 15 mol-% enthält, bei erhöhter Temperatur und bei einer Belastung des Katalysatorfestbetts mit im Reaktionsgasausgangsgemisch enthaltenem Propen von ≥120 NI/I•h so durch ein Katalysatorfestbett, dessen Katalysatoren ringförmige Vollkatalysatoren sind, deren Aktivmasse wenigstens ein Multimetalloxid der allgemeinen Formel I,
Mo₁₂WₐCo_{b}Fe_{c}Bi_{d}SiₑK_{f}Oₙ (I),
ist, mit
a = ≥1 bis ≤3,
b = ≥3 bis ≤8,
c = ≥1 bis ≤4,
d = ≥0,5 bis ≤1,5,
e = ≥0 bis ≤10,
f = ≥0 bis ≤0,2 und
n = eine Zahl, die durch die Wertigkeit und Häufigkeit der von Sauerstoff ver- schiedenen Elemente in I bestimmt wird,
führt, dass der Propenumsatz bei einmaligem Durchgang ≥90 mol-% und die damit einhergehende Selektivität der Acroleinbildung ≥80 mol-% betragen, **dadurch gekennzeichnet,**
**dass** in der Multimetalloxidmasse I zusätzlich die nachfolgenden molaren Verhältnisse erfüllt sind: Co/Fe = 2 bis 4 und Co/Mo = 0,5 bis 0,7.

2. Verfahren nach Anspruch 1, **dadurch gekennzeichnet, dass** der stöchiometrische Koeffizient a 1,5 bis 2,5 beträgt.

3. Verfahren nach Anspruch 1 oder 2, **dadurch gekennzeichnet, dass** der stöchiometrische Koeffizient b ≥5 und ≥8 beträgt.

4. Verfahren nach einem der Ansprüche 1 bis 3, **dadurch gekennzeichnet, dass** der stöchiometrische Koeffizient c ≥2 und ≤4 beträgt.

5. Verfahren nach einem der Ansprüche 1 bis 4, **dadurch gekennzeichnet, dass** das molare Verhältnis Co/Fe 2 bis 3,5 beträgt.

6. Verfahren nach einem der Ansprüche 1 bis 5, **dadurch gekennzeichnet, dass** die ringförmigen Vollkatalysatoren eine ringförmige Geometrie mit einer Länge von 2 bis 11 mm, einem Außendurchmesser von 2 bis 11 mm und einer Wandstärke von 0,5 bis 5 mm aufweisen.

7. Verfahren nach einem der Ansprüche 1 bis 5, **dadurch gekennzeichnet, dass** die ringförmigen Vollkatalysatoren eine ringförmige Geometrie mit einer Länge von 2,8 bis 3,2 mm, einem Außendurchmesser von 5,5 bis 7 mm und einem Innendurchmesser von 3,5 bis 5 mm aufweisen.

8. Verfahren nach einem der Ansprüche 1 bis 7, **dadurch gekennzeichnet, dass** das Reaktionsgasausgangsgemisch 7 bis 15 Vol.-% Propen enthält.

9. Verfahren nach einem der Ansprüche 1 bis 7, **dadurch gekennzeichnet, dass** das Reaktionsgasausgangsgemisch 8 bis 12 Vol.-% Propen enthält.

10. Verfahren nach einem der Ansprüche 1 bis 7, **dadurch gekennzeichnet, dass** das Reaktionsgasausgangsgemisch 5 bis 8 Vol.-% Propen enthält.

11. Verfahren nach einem der Ansprüche 1 bis 10, **dadurch gekennzeichnet, dass** die Belastung des Katalysatorfestbetts mit im Reaktionsgasausgangsgemisch enthaltenem Propen ≥130 NI/I•h beträgt.

12. Verfahren nach einem der Ansprüche 1 bis 10, **dadurch gekennzeichnet, dass** die Belastung des Katalysatorfestbetts mit im Reaktionsgasausgangsgemisch enthaltenem Propen ≥140 NI/I•h beträgt.

13. Verfahren nach einem der Ansprüche 1 bis 12, **dadurch gekennzeichnet, dass** die Aktivmasse ein Multimetalloxid der allgemeinen Formel I ist, das abgegrenzte Bereiche der chemischen Zusammensetzung II,
Bi_{a'}W_{b'}O_{x'} (II),
mit
a' = 0,01 bis 8,
b' = 0,1 bis 30 und
x' = eine Zahl, die durch die Wertigkeit und Häufigkeit der von Sauerstoff verschiedenen Elemente in II bestimmt wird,
enthält.

14. Ringförmiger Vollkatalysator, dessen Aktivmasse ein Metalloxid der allgemeinen Formel I,
MO₁₂WₐCo_{b}Fe_{c}Bi_{d}SiₑK_{f}Oₙ (I),
ist, mit
a = ≥1 bis ≤3,
b = ≥3 bis ≤8,
c= ≥1 bis ≥4,
d = ≥0,5 bis ≤1,5,
e = ≥0 bis ≤10,
f = ≥0 bis ≤0,2 und
n = eine Zahl, die durch die Wertigkeit und Häufigkeit der von Sauerstoff ver- schiedenen Elemente in I bestimmt wird,
mit der Maßgabe, dass in der Multimetalloxidmasse I zusätzlich die nachfolgenden molaren Verhältnisse erfüllt sind:
Co/Fe = 2 bis 3,5 und
Co/Mo = 0,5 bis 0,7.

15. Ringförmiger Vollkatalysator nach Anspruch 14, mit der Maßgabe, dass der stöchiometrische Koeffizient b ≥5 und ≤8 beträgt.

16. Ringförmiger Vollkatalysator nach Anspruch 14 oder 15, mit der Maßgabe, dass der stöchiometrische Koeffizient c ≥2 und ≤4 beträgt.

17. Ringförmiger Vollkatalysator nach einem der Ansprüche 14 bis 16, mit der Maßgabe, dass seine ringförmige Geometrie eine Länge von 2 bis 11 mm, einen Außendurchmesser von 2 bis 11 mm und eine Wandstärke von 0,5 bis 5 mm aufweist.

18. Ringförmiger Vollkatalysator nach einem der Ansprüche 14 bis 16, mit der Maßgabe, dass seine ringförmige Geometrie eine Länge von 2,8 bis 3,2 mm, einen Außendurchmesser von 5,5 bis 7 mm und einen Innendurchmesser von 3,5 bis 5 mm aufweist.

## Claims

1. A process for preparing acrolein by heterogeneously catalyzed partial gas phase oxidation, by conducting a starting reaction gas mixture which comprises propene, molecular oxygen and at least one inert gas and comprises the molecular oxygen and the propene in a molar O₂ : C₃H₆ ratio of ≥ 1, and also carbon dioxide and saturated hydrocarbons together in a total amount of at most 15 mol%, at elevated temperature and at an hourly space velocity on the fixed catalyst bed of propene present in the starting reaction gas mixture of ≥ 120 1 (STP)/1·h through a fixed catalyst bed whose catalysts are annular unsupported catalysts whose active composition is at least one multimetal oxide of the general formula I,
Mo₁₂WₐCo_{b}Fe_{c}Bi_{d}SiₑK_{f}Oₙ (I)
where
a = from ≥ 1 to ≥ 3,
b = from ≥ 3 to ≤ 8,
c = from ≥ 1 to ≤ 4,
d = from ≥ 0.5 to ≤ 1.5,
e = from ≥ 0 to ≤ 10,
f = from ≥ 0 to ≤ 0.2 and
n = a number which is determined by the valency and frequency of the elements in I other than oxygen,
in such a way that the propene conversion in single pass is ≥ 90 mol% and the associated selectivity of acrolein formation is ≥ 80 mol%, wherein,
in the multimetal oxide composition I, the following molar ratios are additionally fulfilled:
Co/Fe = from 2 to 4 and Co/Mo = from 0.5 to 0.7.

2. The process according to claim 1, wherein the stoichiometric coefficient a is from 1.5 to 2.5.

3. The process according to claim 1 or 2, wherein the stoichiometric coefficient b is ≥ 5 and ≤ 8.

4. The process according to any of claims 1 to 3, wherein the stoichiometric coefficient c is ≥ 2 and ≤ 4.

5. The process according to any of claims 1 to 4, wherein the molar Co/Fe ratio is from 2 to 3.5.

6. The process according to any of claims 1 to 5, wherein the annular unsupported catalysts have an annular geometry having a length of from 2 to 11 mm, an external diameter of from 2 to 11 mm and a wall thickness of from 0.5 to 5 mm.

7. The process according to any of claims 1 to 5, wherein the annular unsupported catalysts have an annular geometry having a length of from 2.8 to 3.2 mm, an external diameter of from 5.5 to 7 mm and an internal diameter of from 3.5 to 5 mm.

8. The process according to any of claims 1 to 7, wherein the starting reaction gas mixture comprises from 7 to 15% by volume of propene.

9. The process according to any of claims 1 to 7, wherein the starting reaction gas mixture comprises from 8 to 12% by volume of propene.

10. The process according to any of claims 1 to 7, wherein the starting reaction gas mixture comprises from 5 to 8% by volume of propene.

11. The process according to any of claims 1 to 10, wherein the hourly space velocity on the fixed catalyst bed of propene present in the starting reaction gas mixture is ≥ 130 1 (STP)/1·h.

12. The process according to any of claims 1 to 10, wherein the hourly space velocity on the fixed catalyst bed of propene present in the starting reaction gas mixture its ≥ 140 1 (STP)/1·h.

13. The process according to any of claims 1 to 12, wherein the active composition is a multimetal oxide of the general formula I which comprises delimited regions of the chemical composition II
Biₐ,W_{b},Oₓ (II)
where
a' = from 0.01 to 8,
b' = from 0.1 to 30 and
x' = a number which is determined by the valency and frequency of the elements in II other than oxygen.

14. An annular unsupported catalyst whose active composition is a metal oxide of the general formula I,
Mo₁₂WₐCO_{b}Fe_{c}Bi_{d}SiₑK_{f}Oₙ (I)
where
a = from ≥ 1 to ≤ 3,
b = from ≥ 3 to ≥ 8,
c = from ≥ 1 to ≤ 4,
d = from ≥ 0.5 to ≤ 1.5,
e = from ≥ 0 to ≥ 10,
f = from ≥ 0 to ≤ 0.2 and
n = a number which is determined by the valency and frequency of the elements in I other than oxygen,
with the proviso that the following molar ratios are additionally fulfilled in the multimetal oxide composition I:
Co/Fe = from 2 to 3.5 and
Co/Mo = from 0.5 to 0.7.

15. The annular unsupported catalyst according to claim 14, with the proviso that the stoichiometric coefficient b is ≥ 5 and ≤ 8.

16. The annular unsupported catalyst according to claim 14 or 15, with the proviso that the stoichiometric coefficient c is ≥ 2 and ≤ 4.

17. The annular unsupported catalyst according to any of claims 14 to 16, with the proviso that its annular geometry has a length of from 2 to 11 mm, an external diameter of from 2 to 11 mm and a wall thickness of from 0.5 to 5 mm.

18. The annular unsupported catalyst according to any of claims 14 to 16, with the proviso that its annular geometry has a length of from 2.8 to 3.2 mm, an external diameter of from 5.5 to 7 mm and an internal diameter of from 3.5 to 5 mm.

## Revendications

1. Procédé de fabrication d'acroléine par oxydation partielle en phase gazeuse sous catalyse hétérogène, selon lequel un mélange réactionnel gazeux initial contenant du propène, de l'oxygène moléculaire et au moins un gaz inerte, qui contient l'oxygène moléculaire et le propène en un rapport molaire O₂:C₃H₆ ≥ 1, ainsi que du dioxyde de carbone et des hydrocarbures saturés en une quantité totale d'au plus 15 % en moles, traverse, à température élevée et à une charge du lit catalytique fixe avec le propène contenu dans le mélange réactionnel gazeux initial ≥ 120 N1/1·h, un lit catalytique fixe dont les catalyseurs sont des catalyseurs entiers annulaires, dont la masse active est au moins un oxyde de plusieurs métaux de formule générale 1
MO₁₂WₐCO_{b}Fe_{c}Bi_{d}SiₑK_{f}Oₙ (I)
avec
a = ≥ 1 à ≥ 3,
b = ≥ 3 à ≥ 8,
c = ≥ 1 à ≤ 4,
d = ≥ 0, à ≤ 1,5,
e = ≥ 0 à ≤ 10,
f = ≥ 0 à ≤ 0,2 et
n = un nombre déterminé par la valence et la fréquence des éléments différents de l'oxygène dans I,
de manière à ce que la conversion du propène lors d'un passage unique soit ≥ 90 % en moles et la sélectivité qui l'accompagne pour la formation d'acroléine soit ≥ 80 % en moles, **caractérisé en ce que**
les rapports molaires suivants sont également satisfaits dans la masse d'oxyde de plusieurs métaux I : Co/Fe = 2 à 4 et Co/Mo = 0,5 à 0,7.

2. Procédé selon la revendication 1, **caractérisé en ce que** le coefficient stoechiométrique a est de 1,5 à 2,5.

3. Procédé selon la revendication 1 ou 2, **caractérisé en ce que** le coefficient stoechiométrique b est ≥ 5 et ≤ 8.

4. Procédé selon l'une quelconque des revendications 1 à 3, **caractérisé en ce que** le coefficient stoechiométrique c est ≥ 2 et ≤ 4.

5. Procédé selon l'une quelconque des revendications 1 à 4, **caractérisé en ce que** le rapport molaire Co/Fe est de 2 à 3,5.

6. Procédé selon l'une quelconque des revendications 1 à 5, **caractérisé en ce que** les catalyseurs entiers annulaires présentent une géométrie annulaire ayant une longueur de 2 à 11 mm, un diamètre externe de 2 à 11 mm et une épaisseur de paroi de 0,5 à 5 mm.

7. Procédé selon l'une quelconque des revendications 1 à 5, **caractérisé en ce que** les catalyseurs entiers annulaires présentent une géométrie annuaire ayant une longueur de 2,8 à 3,2 mm, un diamètre externe de 5,5 à 7 mm et un diamètre interne de 3,5 à 5 mm.

8. Procédé selon l'une quelconque des revendications 1 à 7, **caractérisé en ce que** le mélange réactionnel gazeux initial contient 7 à 15 % en volume de propène.

9. Procédé selon l'une quelconque des revendications 1 à 7, **caractérisé en ce que** le mélange réactionnel gazeux initial contient 8 à 12 % en volume de propène.

10. Procédé selon l'une quelconque des revendications 1 à 7, **caractérisé en ce que** le mélange réactionnel gazeux initial contient 5 à 8 % en volume de propène.

11. Procédé selon l'une quelconque des revendications 1 à 10, **caractérisé en ce que** la charge du lit catalytique fixe avec le propène contenu dans le mélange réactionnel gazeux initial est ≥ 130 N1/1·h.

12. Procédé selon l'une quelconque des revendications 1 à 10, **caractérisé en ce que** la charge du lit catalytique fixe avec le propène contenu dans le mélange réactionnel gazeux initial est ≥ 140 N1/1·h.

13. Procédé selon l'une quelconque des revendications 1 à 12, **caractérisé en ce que** la masse active est un oxyde de plusieurs métaux de formule générale I qui contient des domaines limités de composition chimique II
Biₐ,W_{b},Oₓ, (II)
avec
a' = 0,01 à 8,
b' = 0,1 à 30 et
x' = un nombre déterminé par la valence et la fréquence des éléments différents de l'oxygène dans II.

14. Catalyseur entier annulaire, dont la masse active est un oxyde métallique de formule générale I
Mo₁₂WₐCO_{b}Fe_{c}Bi_{d}SiₑK_{f}Oₙ (I)
avec
a = ≥ 1 à ≤ 3,
b = ≥ 3 à ≤ 8,
c = ≥ 1 à ≤ 4,
d = ≥ 0,5 à ≤ 1, 5,
e = ≥ 0 à ≤ 10,
f = ≥ 0 à ≤ 0,2 et
n = un nombre déterminé par la valence et la fréquence des éléments différents de l'oxygène dans I,
à condition que les rapports molaires suivants soient également satisfaits dans la masse d'oxyde de plusieurs métaux I :
Co/Fe = 2 à 3,5 et
Co/Mo = 0,5 à 0,7.

15. Catalyseur entier annulaire selon la revendication 14, à condition que le coefficient stoechiométrique b soit ≥ 5 et ≤ 8.

16. Catalyseur entier annulaire selon la revendication 14 ou 15, à condition que le coefficient stoechiométrique c soit ≥ 2 et ≤ 4.

17. Catalyseur entier annulaire selon l'une quelconque des revendications 14 à 16, à condition que sa géométrie annulaire présente une longueur de 2 à 11 mm, un diamètre externe de 2 à 11 mm et une épaisseur de paroi de 0,5 à 5 mm.

18. Catalyseur entier annulaire selon l'une quelconque des revendications 14 à 16, à condition que sa géométrie annulaire présente une longueur de 2,8 à 3,2 mm, un diamètre externe de 5,5 à 7 mm et un diamètre interne de 3,5 à 5 mm.
